Europäisches Patentamt

(19) European Patent Office

Office européen des brevets

(11) Publication number: **0 231 275 B1**

(12) **EUROPEAN PATENT SPECIFICATION**

(45) Date of publication of patent specification: **02.09.92** (51) Int. Cl.⁵: **D21C 9/00,** C02F 3/34, C12N 9/02

(21) Application number: **86904676.3**

(22) Date of filing: **11.07.86**

(86) International application number: **PCT/US86/01476**

(87) International publication number: **WO 87/00564 (29.01.87 87/03)**

(54) Use of ligninolytic enzymes in paper pulp production.

(30) Priority: **15.07.85 US 755243**
**15.07.85 US 755244**
**15.07.85 US 755245**
**28.03.86 US 845654**
**28.03.86 US 845656**
**28.03.86 US 845657**

(43) Date of publication of application:
**12.08.87 Bulletin 87/33**

(45) Publication of the grant of the patent:
**02.09.92 Bulletin 92/36**

(84) Designated Contracting States:
**AT BE CH DE FR GB IT LI LU NL SE**

(56) References cited:

**TAPPI Journal, vol. 67, no. 10, October 1984 (Atlanta, GA, US) R. Farrel: "Biocatalysts hold promise of better pulp quality", pages 31,33**

(73) Proprietor: **REPLIGEN CORPORATION**
**One Kendall Square Building 700**
**Cambridge, MA 02139(US)**

(72) Inventor: **FARRELL, Roberta**
**177 Hobart Street**
**Danvers, MA 01923(US)**

(74) Representative: **D'haemer, Jan Constant**
**Sandoz Technology Ltd. Patents and Trademarks Division**
**CH-4002 Basle(CH)**

TAPPI, vol. 64, no. 6, published June 1981 (Atlanta, GA, US) "Potential applications of Lignin biodegradation systems", pages 25-27, 29, 31.

TAPPI, vol. 65, no. 6, June 1982 (Atlanta, GA, US) L. Pilon et al.: "Increasing water retention of mechanical pulp by biological treatments", pages 93-96

Biotechnical Advances, vol. 2, no. 2, published 1984 (Oxford, GB) T.W. Joyce et al.: "A continuous biological process to decolorize bleach plant effluents", pages 301-308

Chemical Abstracts, vol. 104, no. 15, 14 April 1986 (Colombus, Ohio, US) T.K. Kirk et al.: "Lignin degrading activity of phanerochaete chrysosporium burds: comparison of cellulase-negative and other strains", pages 374-375, abstract no. 126286g

Proceedings of the National Academy of Sciences of USA, vol. 81, April 1984 (Washington, US) M. Tien et al.: "Lignin-degrading enzyme from phanerochaete chrysosporium: purification, characterization and catalytic properties of a unique H2O2-requiring oxygenase", pages 2280-2284

Science, vol. 221, no. 4611, 12 August 1983 (Lancaster, PA, US) M. Tien et al.: "Lignin-degrading enzyme from the hymenomycete phanerochaete chrysosporium burds", pages 661-663

## Description

The primary chemical method for making pulp from wood involves the digestion of lignin in the wood with sodium sulfide and sodium hydroxide. This is termed the sulfate or kraft process.

Wood pulp produced in the kraft process generally contains 5-8 % by weight of residual, modified lignin which gives the pulp a characteristic brown color. To obtain a pulp of very high brightness and brightness stability, the lignin must be removed by certain oxidizing agents commonly referred to as bleaching chemicals. Many bleaching processes exist but almost all begin with the chlorination extraction (C-E) stage. There is a loss of cellulosic fibers during the C-E stage. The C-E effluents resulting from treated pulp contain a very large number of organic compounds having a bound chlorine content of 2.5-3.5 kg/ton pulp. Some of these compounds, primarily the chlorinated phenolics, are known to have toxic, mutagenic and carcinogenic effects. (Alberti, B.N. and Klibanov, A.M. (1981) Biotechnology and Bioengineering Symp. 11:373-379). These effluents are highly unsuited for recycling within the pulping system due to their high level of corrosive chlorides. Alternatives to chlorine bleaching have, therefore, long been sought by industry.

Hydrogen peroxide has been shown to delignify sulfite pulps satisfactorily, but on its own it is a relatively ineffective means of bleaching kraft pulp. When used in sequences with chlorine-containing bleaching agents, however, peroxide contributes significantly to delignification, pulp brightness and brightness stability.

Oxygen and ozone have been extensively studied for incorporation into the bleaching processes. The major disadvantage of these compounds is their non-specific oxidative attack on cellulosic fibers. Lower pulp yields tend to result and the pulp properties are generally inferior to those obtained with chlorine bleaching sequencing.

Research sponsored by the U.S. Department of Agriculture's (USDA) Forest Products Laboratory has demonstrated that 50-75 % of the residual lignin was removed by fungal cultures of Phanerochaete chrysosporium in 6 to 8 days. Longer incubation resulted in greater lignin reductions, but the data were not quantified. During incubation, the pulp became substantially lighter in color (Kirk, T.K. and Chang, H. (1981) Enzyme Microb. Technol. 3:189-196).

Bleaching is impractically slow using whole fungal cultures. It was found that lignin removal (i.e., kappa number decrease) from kraft pulp followed a triphasic pattern: 1) no lignin removal during establishment of the fungus in the pulp over the first two days, 2) rapid deligninification during the following two days, and 3) slower deligninification thereafter. The initial two-day lag is due to the secondary metabolic importance of lignin degradation to fungal growth.

Another disadvantage of fungal bleaching is that these organisms contain enzymes which degrade both cellulose and hemicellulose. In any effective bleaching scheme, the degradation of cellulosic fibers must be completely suppressed, since the cellulosic fibers are particularly vulnerable after kraft pulping. Cellulase-less mutants have to some extent overcome this problem, but they are difficult to manage and some are less efficient in degrading lignin than normal fungal cultures. A final disadvantage of using fungal cells is that they can only operate optimally in an environment where temperature and microbial contamination are carefully controlled.

The objective of mechanical pulping is to produce high-yielded pulps. Several years ago mechanical pulping was limited to a single process, the grinding of round-wood against a pulpstone, but since then mechanical pulping has expanded into an array of processes that use chemical, thermal and compression technologies (Casey, J.P. (1983) Tappi Journal 66:95-96). A drawback to the current methods used is that they produce pulp with poor bonding strength and poor brightness stability.

Thermomechanical pulp (TMP), chemithermomechanical pulp (CTMP) and chemimechanical pulp (CMP) processes have evolved to improve mechanical pulp quality, expanding its utility in end product applications. Thermomechanical pulping is the dominant alternative high-yield pulping process. Its major limitation is the requirement for high electrical energy input, most of which ends up as low grade heat.

The utilization of thermomechanical pulps would be greatly facilitated if there was an increase of strength properties and if the stability of brightening could be enhanced, i.e., prevent brightness reversion. Brightness reversion of commercial pulps can be related to the presence of oxidized groups. These groups are principally derived from the residual breakdown products of lignin. It is postulated that the introduction of aldehyde and ketone groups into cellulose upon bleaching also contributes to brightness reversion, although to a lesser extent (Springer, E.L. (1983) Tappi Journal 66:93-96). Breakdown products of lignin cause brightness reversion by mechanisms that are now being elucidated in several laboratories. It has been postulated that $\alpha$-carboxyl groups adjacent to aromatic rings in residual lignin absorb daylight and transfer this energy to oxygen which in turn reacts with the phenolic groups of the lignin leading to formation of colored (yellow) quinones (Rapson, W.H. (1969) Appita 23:102-114). This reaction can occur only on "exposed" lignin rings which contain a free hydroxyl group.

Coarse TMP can be produced with relatively low energy input. Subsequent secondary refining, however, requires substantial energy for development of pulp properties (Higuchi, T. (1982) Experientia 38:159-166). Experiments have demonstrated (Pilon, L., Desrochers, M., Jurasek, L., Neumann, P.J. (1982) Tappi Journal 65:93-96) that treatment of coarse TMP with P. chrysosporium cultures for 14 days can substantially reduce the energy requirement for secondary refining without a loss in pulp quality. Preliminary studies showed that the energy requirements to develop a given freeness in fungal-treated pulp was reduced by 25-30 % as compared to untreated pulps. Furthermore, pulp properties, as measured by the burst index, were also improved considerably. Because the refining of mechanical pulps after swelling in alkali can considerably improve strength properties, both the fungus-treated and untreated pulps were subjected to refining after swelling in alkali. The fungus-treated pulp then required 50 % less refining energy than did the untreated pulp without any loss in strength properties.

The technical problems in applying organisms to industrial mechanical pulps, including TMP processing, are threefold: a) in scaling-up with the required careful control of humidity, aeration and temperature; b) in preventing contamination by unwanted organisms; and (c) in the impractical slowness of lignin degradation.

The primary chemical method for making pulp from wood involves the digestion of lignin in the wood with sodium sulfide and sodium hydroxide. This is termed the sulfate or kraft process.

Wood pulp produced in the kraft process generally contains 5-8 % by weight of residual, modified lignin which gives pulp a characteristic brown color. To obtain pulp of very high brightness and brightness stability, the lignin must be removed by certain oxidizing agents commonly referred to as bleaching chemicals. Many bleaching processes exist but almost all begin with the chlorination-extraction (C-E) stage. The spent liquor from the first alkali extraction stage of bleaching following chlorination, commonly referred to as $E_1$ effluent, contains over 80 % of the effluent color emanating from a kraft bleach plant (Kirk, T.K. and Chang, H-M. (1981) Enzyme Microb. Technol. 3:189-196). The effluent must be discharged due to its high content of corrosive chlorides. Polymeric lignin degradation products, the main contributors to color of bleach plant effluent, are resistant to the current bacteria-based effluent treatment process. Alternate treatment processes such as ultrafiltration, carbon adsorption, and massive lime precipitation are required for effective color removal, but are quite expensive. Economical color removal systems do not presently exist and would be desirable for effluent treatment prior to its discharge to receiving waters.

Fungal decolorization systems have been studied. In USDA sponsored laboratory experiments (Kirk, T.K. (1983) in The Filamentous Fungi, Vol. 4, Fungal Technology, Smith, J.E., Berry, D.R., Kristiansen, B., eds., Edward Arnold Press, London), greater than 80 % decolorization of bleaching effluent prepared by chlorination and alkali treatment of kraft-cooked synthetic lignins has been achieved in 24 hours using Phanerochaete chrysosporium cultures.

There are three problems in using fungal cultures to decolorize bleach plant effluents: (1) fungi require careful culture conditions (i.e., humidity, aeration, temperature and pH) not compatible with industrial processing environments; (2) fungi require long lag times and then only very slowly degrade lignin; and (3) fungi cannot grow on lignin. An additional food source must be added to support fungal growth.

In TAPPI journal, vol. 67, No. 10, October 1984 the inventor of the current patent application discusses the possible use of Phanerochaete chrysosporium enzymes in bleaching, pulping and effluent - decolorisation. However, there is no disclosure therein that the lignolytic mixture used to bleach the kraft pulp, treat the mechanical pulp, or decolorise $E_1$ effluent is harvested from a mutant culture of Phanerochaete chrysosporium by ultrafiltration through a 10 K filter.

The subject invention provides a process selected from:
a) bleaching kraft pulp; or
b) enhancing the strength properties and brightness stability of mechanical pulp; or
c) decolorising $E_1$ effluent,
which process comprises treating said kraft pulp, or mechanical pulp, or $E_1$ effluent with a Ligninolytic Mixture which is a concentrate of the extracellular growth medium from cultures of Phanerochaete chrysosporium, characterised in that the concentrate is obtained from a mutant culture having the identifying characteristics of NRRL 15978 and that the extracellular growth medium is harvested from a culture of the mutant strain by centrifugation and concentrated by ultrafiltration through a 10 K filter. The Ligninolytic Mixture contains ligninases which are highly specific and which will degrade the hard-to-remove residual lignin polymers in chemical pulps without damaging cellulosic fibers.

The said ligninases can bleach kraft pulp and they are immediately active. Thus, there is no lag in activity as with fungal cultures. Since the ligninases are biological molecules, they are, advantageously, not corrosive, do not cause pollution, and do not present an environmental hazard when released.

The subject invention also concerns the enhancement of the strength properties of mechanical pulps, including TMP, CTMP, and CMP, by treating them with the said ligninolytic mixture. The ligninolytic enzymes present in the said extracellular growth medium selectively degrade only the chemical moieties formed in lignin and will not degrade cellulose or hemicellulose.

The ligninolytic mixture can enhance the strength properties of these pulps and the enzymes therein are immediately active. Thus, there is no lag in activity as with fungal cultures. Since the ligninolytic enzymes are biological molecules, they are, advantageously, not corrosive, do not cause pollution and do not present an environmental hazard when released.

As indicated above, the subject invention also concerns the decolorization of $E_1$ effluent by treating the effluent with a ligninolytic mixture in accordance with claim 1 appended hetero.

The ligninolytic mixture which can be used in the subject invention process was isolated from a novel stable mutant strain of the white-rot fungus Phanerochaete chrysosporium. The novel mutant strain, designated SC26, has been deposited in the permanent collection of a public culture repository, to be maintained for at least 30 years. The culture repository is the Northern Regional Research Laboratory, U.S. Department of Agriculture, Peoria, Illinois 61604, USA. The accession number is NRRL 15978, and the deposit date is July 3, 1985. This deposited culture is available to the public as required by patent laws in countries wherein counterparts of the subject application, or its progeny, are filed. However, it should be understood that the availability of a deposit does not constitute a license to practice the subject invention in derogation of patent rights granted by governmental action.

Novel mutant SC26 was obtained by UV mutagenesis of the wild type Phanerochaete chrysosporium, ATCC 24725.

Novel mutant SC26 was grown on a nitrogen-limited trace element medium supplemented with glucose and buffered at pH 4.5.

Isolation and purification of the ligninases from the extracellular fluid in the fermentation was accomplished by ultrafiltration and fast protein liquid chromatography (FPLC) using an anion exchange column.

The Ligninolytic Mixture used in the subject invention process was prepared as follows:

Preparative Example 1--Growth of Mutant SC26 (NRRL 15978) to Produce Fermentation Medium Containing Novel Ligninases

Inoculum was prepared by homogenizing 50 ml of 1.5 day cultures of mutant SC26 grown in 1 liter flasks containing the following medium, designated nitrogen-limited BIII/glucose medium:

The BIII medium contains $1.08 \times 10^{-3}$ M ammonium tartrate, $1.47 \times 10^{-2}$ M $KH_2PO_4$, $2.03 \times 10^{-3}$ M $MgSO_4.7H_2O$, $6.8 \times 10^{-4}$ M $CaCl_2.2H_2O$, $2.96 \times 10^{-6}$ M thiamine.HCl and 10 ml . $L^{-1}$ of a trace element solution. The trace element solution contains $7.8 \times 10^{-3}$ M nitriloacetic acid, $1.2 \times 10^{-2}$ M $MgSO_4.7H_2O$, $1.7 \times 10^{-2}$ M NaCl, $3.59 \times 10^{-4}$ M $FeSO_4.7H_2O$, $7.75 \times 10^{-4}$ M $CoCl_2$, $9.0 \times 10^{-4}$ M $CaCl_2$, $3.48 \times 10^{-4}$ M $ZnSO_4.7H_2O$, $4 \times 10^{-5}$ M $CuSO_4.5H_2O$, $2.1 \times 10^{-5}$ M $AlK(SO_4)_2.12H_2O$, $1.6 \times 10^{-4}$ M $H_3BO_3$, $4.1 \times 10^{-5}$ M $NaMoO_4.2H_2O$ and $2.9 \times 10^{-3}$ M $MnSO_4.H_2O$.

The medium was supplemented with 10 % (by wt/liter) of glucose. The medium was buffered with 10 mM transaconitic acid, pH 4.5

Flasks (125 ml, containing 10 ml sterile medium having the above-described medium) were each inoculated with 0.5 ml of the above homogenate and kept stationary at 39°C. The flasks were flushed on days 0, 3, and 6 with water-saturated $O_2$. Alternatively, a rotating biological contractor (RBC) was used to grow the fungus. 2.5 liters of the above-described medium was inoculated with 100 ml of the above homogenate and grown at 39°C with the RBC rotating at 1 rpm with continuous oxygenation. Ligninase activity was measured periodically by determining the rate of oxidation of veratryl alcohol to veratrylaldehyde. Reaction mixtures contained 275 $\mu$l of extracellular fluid (from flasks or the RBC), 2 mM veratryl alcohol, 0.4 mM $H_2O_2$ addition immediately after buffer was added and were monitored at 310 nm. Protein was determined according to Bradford (Bradford, M.M. (1976) Anal.Biochem. 72:248-254) using bovine serum albumin (Sigma Chemical, St. Louis, MO) as standard.

Preparative Example 2--Isolation and Purification of the Novel Ligninolytic Enzymes

The extracellular growth media from cultures grown in flasks, as described above, was harvested by centrifugation at 5000 xG, 10 min., 4°C. Extracellular growth media was then concentrated by ultrafiltration through a 10 K filter. The resulting concentrate is called the Ligninolytic Mixture ™. The Ligninolytic Mixture ™ can contain one or more of rLDM ™s (ligninolytic enzymes described and characterised hereinafter) or other ligninolytic enzymes in varying proportions. The rLDM ™ contained in this Ligninolytic Mixture ™ were separated by fast protein liquid chromatography (FPLC) using a Pharmacia Mono Q column (Pharmacia, Piscataway, NJ) and a gradient of sodium acetate buffer, pH 6, from 10 mM to 1 M. rLDM ™ 1, 2, 3, 4, 5, and 6 elute from the column in a typical preparation at the following sodium acetate molarities, respectively: 0.16, 0.1818, 0.34, 0.40, 0.58, and 0.43 M to give essentially pure rLDM ™ 1-6. Each rLDM ™ is substantially free of other rLDM ™ and native proteins including substantial freedom from undesirable native destructive proteases. There are indications of these proteases in crude mixtures which are difficult to separate (each substantially pure rLDM ™ gives a negative result in the Azocoll test).

Characterization of the Novel rLDM ™

The rLDM ™ have been characterized by the following criteria:
(1) ability to catalyze the oxidation of veratryl alcohol to veratrylaldehyde;
(2) molecular weight as determined by SDS-PAGE;
(3) amino acid composition;
(4) heme content;
(5) homology by antibody reactivity;
(6) specificity of activity against lignin model substrates; and
(7) elution from an FPLC column at specified acetate molarities.

All of the rLDM ™ catalyze the oxidation of veratryl alcohol to veratrylaldehyde, as monitored spectrophotometrically at 310 nm. A unit of activity is defined as the production of 1 micromole of veratryl-aldehyde in the rLDM ™ catalyzed reaction. The specific activities of typical preparations at about 24°C are as follows:

| rLDM ™ | 1 | 2 | 3 | 4 | 5 | 6 |
|---|---|---|---|---|---|---|
| Specific Activity Units/MG.Minutes | 2.6 | 17.1 | 5.1 | 9.7 | 9.4 | 12.4 |
| Molecular Weight kD | 38 | 38 | 42 | 42 | 43 | 42 |

Amino acid composition--Amino acid composition was determined by a modification of the procedure of Jones et al. (Jones, B.N., Paabo, S. and Stein, S. (1981) J. Liquid Chromatography 4:565-586). The ratio of amino acids is approximately due to the limitation of technique and quantity of protein used in the determination. See Table 1

Heme and carbohydrate content--rLDM ™ 1, 2, 3, 4, 5, and 6 each contain a single protoheme IX Moiety. All are glycosylated according to periodic acid staining (PAS) and binding to Con A-Sepharose (Sigma).

Immunoblot Procedure

This procedure was used to further characterize the rLDM ™. It is a standard procedure which is disclosed in Towbin et al. (Towbin, H., Staehelin, T. and Gordon, J. (1979) Proc. Natl. Acad. Sci. USA 76:4350). The procedure involves separating the proteins by electrophoresis in a gel, transfer of the proteins to a solid matrix, and reacting with (1) a primary probe, rabbit anti-rLDM ™ antibody and (2) a secondary probe, goat anti-rabbit antibody coupled to horseradish peroxidase.

rLDM ™ 1, 2, 3, 4, 5, and 6 react to polyclonal antibodies made to rLDM ™ 2 and 6, using the above immunoblot procedure. rLDM ™ 2, in the same procedure, reacts to polyclonal antibodies made to rLDM ™ 6.

All the rLDM ™ disclosed herein have the following unique activities on lignin substrates, i.e., veratryl alcohol, 1-(3',4'-dimethoxyphenyl)-glycerol-$\beta$-guaiacyl ether, phenol, methoxylated benzenes such as 1,4-dimethoxybenzene:
(1) oxidative cleavage of $C_\alpha$ - $C_\beta$ ;
(2) hydroxylation of benzylic methylene groups;
(3) oxidation of benzyl alcohols to aldehydes;
(4) phenol oxidation; and
(5) oxidation of methoxy and ethoxy benzene.

"Lignin model substrates" are chemicals which resemble parts of lignin. The reaction products of the model compounds with rLDM ™s can have practical utility particularly to but not limited to food, pharmaceutical and chemical industries as chemical feedstocks. The above activities are characteristic of the rLDM ™ disclosed herein.

Following are Examples which illutrate the best mode for practicing the invention. These Examples should not be construed as limiting. In all Examples herein, percentages are by weight and solvent mixture proportions are by volume unless otherwise noted.

### Example 1--Bleaching of Kraft Pulp with rLDM ™ and Other Ligninolytic Enzymes

The Ligninolytic Mixture ™, as described in Preparative Example 2, was added to kraft pulp having a characteristic brown color at 3 % consistency in 10 mM trans-aconitic acid, pH 4.5, 400 $\mu$M $H_2O_2$ and 100 $\mu$M $MnSO_4$. The pulp slurry was flushed with $O_2$ and incubated with slow shaking at 39°C for 12 hr, after which the kraft pulp solution was decanted, and 1 M NaOH solution was added to the pulp and incubated for 60 min at 65°C. This was then decanted and the kraft pulp was washed in water. The resulting kraft pulp no longer had a dark brown color, but instead had a desired lighter color.

The use of $MnSO_4$ is optional.

Regarding the above conditions, for each of the parameters there is a range of values which can be used to achieve the desired result. Typical values and acceptable ranges for each parameter are shown in Table 2.

### Example 2

Upon substituting the Ligninolytic Mixture ™ of Example 1 with extracellular growth medium, prepared as disclosed in Preparative Example 1, there is obtained kraft pulp having a desired lighter brown color.

### Example 3

Upon substituting the Ligninolytic Mixture ™ of Example 1 with a mixture comprising all of the following or any combination thereof: rLDM ™ 1-6, individually or mixtures thereof; Ligninolytic Mixture ™; and extracellular growth medium; there is obtained kraft pulp having a desired lighter brown color.

### Example 4--Treatment of TMP with rLDM ™ and Other Ligninolytic Enzymes

The Ligninolytic Mixture ™, as described in Preparative Example 2, (0.15-1.5 mg protein total) was added to 10 gm of TMP (dry weight) at 3 % consistency in 10 mM trans-aconitic acid, pH 4.5, 400 $\mu$M $H_2O_2$ and 100 $\mu$M $MnSO_4$. The pulp slurry was flushed with $O_2$ and incubated with slow shaking at 39°C for 12 hr, after which time the TMP was washed with water. The tensile, tear and burst indices as well as breaking length of the pulp was measured and found to be of enhanced strength versus an untreated sample. The brightness reversion of the treated sample was less than that of the untreated sample; therefore, brightness stability was increased with the Ligninolytic Mixture ™ treatment.

The use of $MnSO_4$ is optional.

Regarding the above conditions, for each of the parameters there is a range of values which can be used to achieve the desired result. Typical values and acceptable ranges for each parameter are shown in Table 3.

### Example 5

Upon substituting the Ligninolytic Mixture ™ of Example 4 with extracellular growth medium, prepared as disclosed in Preparative Example 1, there is obtained pulp of high quality.

### Example 6

Upon substituting the Ligninolytic Mixture ™ of Example 4 with a mixture comprising all of the following or any combination thereof: rLDM ™ 1-6, individually or mixtures thereof; Ligninolytic Mixture ™; and extracellular growth medium; there is obtained pulp of high quality.

### Example 7

Upon substituting CTMP or CMP for the TMP in Examples 4-6 there is obtained pulp of high quality.

### Example 8--Decolorization of Effluent with rLDM ™ and Other Ligninolytic Enzymes

The Ligninolytic Mixture ™, as described in Preparative Example 2, was added to a 0.2 % solution of $E_1$ effluent in 10 mM trans-aconitic acid, pH 4.5, 400 $\mu$M $H_2O_2$ and 100 $\mu$M $MnSO_4$. The solution was flushed with $O_2$ and incubated with slow shaking at 39°C for 12 hr. The solution was monitored spectrophotometrically in the ultraviolet and visible regions. $E_1$ effluent treated as above was noticeably decolorized and reduced in absorbance at 465 nm. (Note that color is measured by A465 nm wherein an absorbance of 1.0 at 465 nm, pH 7.6 equals 3774 National Council for Air and Stream Improvement color units.)

The $MnSO_4$ is optional.

Regarding the above conditions, for each of the parameters there is a range of values which can be used to achieve the desired result. Typical values and acceptable ranges for each parameter are shown in Table 4.

Example 9

Upon substituting extracellular growth medium from a Phanerochaete chrysosporium fermentation, obtained as disclosed in Preparative Example 1, for the Ligninolytic Mixture ™ of Example 8, there is obtained decolorized $E_1$ effluent.

Example 10

Upon substituting the Ligninolytic Mixture ™ of Example 8 with a mixture comprising all of the following or any combination thereof: rLDM ™ 1-6, individually or mixtures thereof; Ligninolytic Mixture ™; and extracellular growth medium; there is obtained decolorized $E_1$ effluent.

## Table I
## Amino Acid Composition of rLDM$^{TM}$

| Amino Acid | rLDM$^{TM}$ 1 | rLDM$^{TM}$ 2 | rLDM$^{TM}$ 3 | rLDM$^{TM}$ 5 | rLDM$^{TM}$ 6 |
|---|---|---|---|---|---|
| | Ratio | Ratio | Ratio | Ratio | Ratio |
| asp/asn | 1.4 | 2.0 | 5.4 | 5.0 | 3.0 |
| glu/gln | 6.0 | 7.7 | 16.8 | 19.9 | 8.0 |
| ser | 4.3 | 4.1 | 14.0 | 22.3 | 6.8 |
| his | 4.4 | 3.2 | 7.3 | 15.3 | 3.2 |
| gly | 6.5 | 5.7 | 24.0 | 44.7 | 8.3 |
| thr | 2.2 | 3.5 | - | - | 4.9 |
| arg | 1.1 | 1.2 | 2.9 | 4.8 | 1.3 |
| ala | 7.3 | 7.9 | 14.4 | 13.8 | 6.7 |
| tyr | 0.2 | - | 1.0 | 1.0 | 0.2 |
| met | - | - | 1.2 | - | 0.14 |
| val | 1.6 | 2.6 | 7.4 | 6.5 | 4.2 |
| phe | 1.1 | 3.0 | 7.0 | 3.3 | 3.2 |
| ile | 1.0 | 2.2 | 4.1 | 3.6 | 2.4 |
| leu | 1.5 | 2.6 | 6.5 | 6.0 | 3.3 |
| lys | 0.5 | 1.0 | 2.5 | 2.3 | 1.0 |

EP 0 231 275 B1

Table 2

| Parameter | Typical | Range |
|---|---|---|
| Consistency | 3% | 0.01 to 20%* |
| Concentration of trans-aconitic acid** | 10 mM | 0.005 to 0.5 M |
| pH | 4.5 | 2 to 7 |
| Concentration of $H_2O_2$ | 400 $\mu$M | 2 $\mu$M to 10 mM |
| Concentration of $MnSO_4$ | 100 $\mu$M | 10 to 500 $\mu$M |
| Incubation of pulp slurry (First incubation) | 12 hr | 2 min to 48 hr |
| Temperature of first incubation | 39°C | 15° to 50°C |
| Concentration of NaOH*** | 1 M | 0.01 to 5 M |
| Incubation of pulp after alkaline treatment (Second incubation) | 60 min | 2 min to 48 hr |
| Temperature of second incubation | 65°C | 5° to 100°C |

*Concentrations greater than 20% can be used if the fluid consistency of the medium is maintained.
**Other nontoxic enzyme buffers such as ammonium tartrate can be used.
***KOH or other alkaline solutions can be used.

Table 3

| Parameter | Typical | Range |
|---|---|---|
| Consistency | 3% | 0.01 to 20%* |
| Ratio of Ligninolytic Mixture ™ to mechanical pulps (mg of protein/g of pulp) | 0.08 | 0.015 to 0.15 |
| Concentration of trans-aconitic acid** | 10 mM | 0.005 to 0.5 M |
| pH | 4.5 | 2 to 7 |
| Concentration of $H_2O_2$ | 400 $\mu$M | 2 $\mu$M to 10 mM |
| Concentration of $MnSO_4$ | 100 $\mu$M | 10 to 500 $\mu$M |
| Incubation period | 12 hr | 2 min to 48 hr |
| Temperature during incubation | 39°C | 15 to 50°C |

*Concentrations greater than 20% can be used if the fluid consistency of the medium is maintained.
**Other nontoxic enzyme buffers such as ammonium tartrate can be used.

Table 4

| Parameter | Typical | Range |
|---|---|---|
| Concentration of effluent | 0.2% | 0.01 to 20% |
| Concentration of trans-aconitic acid* | 10 mM | 0.005 to 0.5 M |
| Concentration of Ligninolytic Mixture ™ | 1 VAO/Unit/ml** | 0.01 to 30 Units/ml |
| pH | 4.5 | 2 to 7 |
| Concentration of $H_2O_2$ | 400 $\mu$M | 2 $\mu$M to 10 mM |
| Concentration of $MnSO_4$ | 100 $\mu$M | 10 to 500 $\mu$M |
| Incubation period | 12 hr | 2 min to 48 hr |
| Temperature during incubation | 39°C | 15° to 50°C |

*Other nontoxic enzyme buffers such as ammonium tartrate can be used.
**VAO/Unit = veratryl alcohol oxidation activity unit

**Claims**

1. A process selected from:

a) bleaching kraft pulp; or

b) enhancing the strenght properties and brightness stability of mechanical pulp; or

c) decolorising E$_1$ effluent,

which process comprises treating said kraft pulp, or mechanical pulp, or E$_1$ effluent with a Ligninolytic Mixture which is a concentrate of the extracellular growth medium from cultures of Phanerochaete chrysosporium, characterised in that the concentrate is obtained from a mutant culture having the identifying characteristics of NRRL 15978 and that the extracellular growth medium is harvested from a culture of the mutant strain by centrifugation and concentrated by ultrafiltration through a 10 K filter.

2. A process according to claim 1, wherein said mechanical pulp is TMP, or CTMP or CMP.

**Patentansprüche**

1. Ein Verfahren aus der Gruppe

a) Bleichen von Kraft Pulpe,

b) Verbessern der Stärkeeigenschaften und der Weissestabilität von mechanischer Pulpe und

c) Entfärben von E$_1$ Abwasser,

das darin besteht, die Kraft Pulpe, mechanische Pulpe oder E$_1$ Abwasser mit einer ligninolytischen Mischung zu behandeln, die ein Konzentrat des extrazellulären Wachstumsmilieu von Phanerochaete chrysosporium Kulturen ist,

dadurch gekennzeichnet, dass das Konzentrat von einer mutanten Kultur mit den Charakteristiken von NRRL 15978 stammt und dass das extrazelluläre Wachstumsmilieu aus der Kultur dieses mutanten Stammes durch Zentrifugieren abgetrennt und durch Ultrafiltration über einen 10 K Filter konzentriert wird.

2. Ein Verfahren gemäss Anspruch 1, dadurch gekennzeichnet, dass die mechanische Pulpe TMP oder CTMP oder CMP ist.

**Revendications**

1. Un procédé choisi parmi :

a) le blanchiment des pâtes kraft,

b) l'augmentation des caractéristiques de résistance et de la stabilité de la blancheur des pâtes mécaniques, et

c) la décoloration des effluents E$_1$,

lequel procédé comprend le traitement des pâtes kraft, des pâtes mécaniques ou des effluents E$_1$ par un Mélange Ligninolytique qui est un concentré du milieu de croissance extracellulaire provenant de cultures de Phanerochaete chrysosporium, caractérisé en ce que le concentré est obtenu à partir d'une culture de mutant ayant les caractéristiques d'identification de NRRL 15978 et que le milieu de croissance extracellulaire est recueilli à partir de la culture de la souche de mutant par centrifugation et est concentré par ultrafiltration sur filtre 10 K.

2. Un procédé selon la revendication 1, dans lequel ladite pâte mécanique est la TMP, la CTMP ou la CMP.